# EUROPEAN PATENT SPECIFICATION

(11) **EP 4 431 141 B1**
(45) Date of publication and mention of the grant of the patent: **15.04.2026**
(21) Application number: 22903264.4
(22) Date of filing: 29.11.2022
(51) Int. Cl.: A61M 16/16, A61M 16/00

(54) **HUMIDIFIER AND VENTILATION TREATMENT APPARATUS**
BEFEUCHTER UND BEATMUNGSBEHANDLUNGSVORRICHTUNG
HUMIDIFICATEUR ET APPAREIL DE TRAITEMENT DE VENTILATION

(30) Priority: 06.12.2021 CN 202111478429
(43) Date of publication of application: 18.09.2024
(73) Proprietor: BMC Medical Co., Ltd., Beijing 100073 (CN)
(72) Inventor: LIU, Lijun, Beijing 100041 (CN); ZHUANG, Zhi, Beijing 100041 (CN)
(74) Representative: Grünecker Patent- und Rechtsanwälte PartG mbB
(86) International application number: PCT/CN2022/135077
(87) International publication number: WO 2023/103842

(56) References cited:
- AU-A1- 2015 200 180
- CN-A- 101 966 363
- CN-A- 101 966 363
- CN-A- 112 426 606
- CN-A- 113 018 631
- CN-A- 114 209 952
- CN-U- 205 460 329
- CN-U- 205 460 329
- CN-U- 215 194 802
- CN-U- 216 908 874
- JP-A- H05 228 214
- US-A1- 2020 069 904

## Description

### FIELD

The present disclosure relates to the field of ventilation treatment, in particular to a humidifier and a ventilation treatment apparatus including the humidifier.

### BACKGROUND

In modern clinical medicine, people pay more and more attention to the comfort during the use of ventilation treatment apparatus. Especially, in the dry winter in some regions, it is necessary to equip ventilation treatment apparatus with humidifiers. The humidified gas generated by a humidifier can moisturize and humidify the nasal cavity, nasal mucosa, respiratory tract, heart, and lung of the patient, thereby the treatment effect on the patient and the compliance of the patient in wearing the ventilator can be improved, and the humidifier is even more important for a patient who wears the ventilator for a long time.

At present, humidifiers for ventilation treatment apparatus available on the market are generally categorized into two types: one type of humidifiers are steam humidifiers, in which water is vaporized by electric heating, and gas at a certain pressure is humidified before reaching the patient side for use; the other type of humidifiers are ultrasonic humidifiers, in which water is vibrated into particles at high frequency by ultrasonic waves, and gas at a certain pressure passes and carries the water particles to the patient side for use.

However, these two types of humidifiers have some disadvantages and limitations:
A steam humidifier has specific requirements for water quality. Purified water or distilled water may be used in the water tank. If other water, such as tap water or mineral water, is used, scale will appear on the bottom or side walls of the water tank after long-time heating, and the scale is not conducive to the cleaning of the water tank, and may enter the respiratory tract of the patient, posing a safety hazard. Moreover, the gas from the outlet of the steam humidifier is at a certain temperature (usually higher than the ambient temperature), and a soft respiratory conduit is usually used to connect the patient side with the ventilator side. When the warm and humid gas at a temperature higher than the ambient temperature passes through the respiratory conduit, condensed water may be formed on the inner wall of the respiratory conduit owing to a temperature difference between the ambient temperature outside the respiratory conduit and the temperature of the warm and humid gas inside the respiratory conduit. The condensed water accumulated during long-time use may lead to blockage of the respiratory conduit, water intrusion into the nasal cavity or respiratory tract of the patient, and failure of the ventilation treatment apparatus resulted from water backflow.

With an ultrasonic humidifier, the water particles produced by high-frequency vibration of ultrasonic waves are relatively large, can be seen with naked eyes, and usually exist in the form of water mist. The large water particles will slowly condense into water droplets on the wall of the respiratory conduit and then into accumulated water, which will lead to the same problems as the condensed water in a steam humidifier.

US 2020/0069904 discloses a non-heated humidification device comprising a wick, a chamber for holding water in contact with the wick, and a gas inlet to the chamber, wherein the chamber and wick are configured to humidify gas passing through or over the wick at ambient conditions. CN 112426606A discloses an oxygen surface humidifying device comprising a main shell and a humidifying unit, at least one air inlet pipe allowing dry oxygen to enter the main shell and at least one air outlet pipe allowing humidified oxygen to flow out are arranged on the main shell, and humidifying liquid is contained in the main shell.

### SUMMARY

The object of the present disclosure is to provide a humidifier and a ventilation treatment apparatus including the humidifier to solve the above problems.

In order to achieve the above object, the present invention provides a humidifier, which comprises a water tank having a water storage cavity, and a water-absorbing and gas-permeable component arranged in the water storage cavity, wherein a part of the water-absorbing and gas-permeable component is configured, in use, to be located below a water level of the water storage cavity; and a gas chamber is defined inside the water-absorbing and gas-permeable component; wherein the humidifier comprises a supporting component, which is arranged in the water storage cavity and used for supporting the water-absorbing and gas-permeable component, and wherein the supporting component is provided with a guiding part for guiding the supporting component into the water storage cavity.

Optionally, the water tank is provided with a gas inlet tube and a gas outlet tube, a gas outlet end of the gas inlet tube is inserted into the gas chamber and is located above the water level of the water storage cavity, and the gas outlet tube is in communication with the water storage cavity outside the water-absorbing and gas-permeable component.

Optionally, the water-absorbing and gas-permeable component comprises a hollow cylinder, an internal cavity of the cylinder is the gas chamber, and a top of the cylinder is provided with an opening for inserting the gas inlet tube.

Optionally, the supporting component comprises a mounting frame and a supporting frame connected above the mounting frame, the supporting component is mounted at a bottom of the water storage cavity via the mounting frame, and the water-absorbing and gas-permeable component is supported on the supporting frame.

Optionally, the mounting frame is in a disk shape matching a circumferential profile of the water storage cavity, the supporting frame is in a cylindrical shape extending upward from the mounting frame, and a bottom of the cylinder is open and sleeved outside the supporting frame.

Optionally, the water-absorbing and gas-permeable component comprises an annular extension extending horizontally outward from a top peripheral edge of the cylinder, an outer circumferential surface of the annular extension is closely attached to a circumferential wall of the water storage cavity, and the gas outlet tube is in communication with the water storage cavity above the annular extension.

Optionally, the mounting frame comprises an inner ring, an outer ring sleeved outside the inner ring at intervals, and a plurality of connecting ribs connected between the inner ring and the outer ring at intervals along a circumferential direction; the supporting frame comprises an upper ring and a lower ring which are arranged at intervals up and down, and a plurality of supporting ribs connected between the upper ring and the lower ring and arranged at intervals along the circumferential direction, wherein:
the lower ring is connected to the inner ring, or the lower ring is the inner ring.

Optionally, the guiding part is a chamfer formed at an outer rim of the outer ring.

Optionally, a bottom end of the cylinder is provided with a plurality of grooves for embedding the connecting ribs at an interval in the circumferential direction, and the plurality of grooves are in one-to-one correspondence with the connecting ribs.

Optionally, a gas inlet end of the gas inlet tube is arranged opposite to a gas outlet end of the gas outlet tube.

Optionally, the water-absorbing and gas-permeable component is made of a fibrous material, cotton or absorbent paper.

Optionally, the water tank comprises a box body with a top opening, a cover body detachably covering the top opening, and a seal arranged between the cover body and the box body.

In another aspect, the present disclosure provides a ventilation treatment apparatus, which comprises the humidifier described above.

With the above technical scheme, an evaporation filter screen is formed from the water-absorbing and gas-permeable component that has excellent water absorbability and gas permeability, a part of the water-absorbing and gas-permeable component is immersed in water in the water storage cavity to absorb water and diffuse moisture to the rest part of the water-absorbing and gas-permeable component, and gas entering the water storage cavity at a certain pressure can flow through the part of the water-absorbing and gas-permeable component that is not immersed in water at a high speed, so that the moisture in the water-absorbing and gas-permeable component can be evaporated and diffused into the water storage cavity rapidly, thereby realizing gas humidification; the technical scheme doesn't require heating, doesn't produce any temperature difference and condensed water, and doesn't involve the problems existing in steam humidifiers and ultrasonic humidifiers.

Other features and advantages of the present disclosure will be further detailed in the following embodiments.

### BRIEF DESCRIPTION OF THE DRAWINGS

The accompanying drawings are provided herein to facilitate further understanding on the present disclosure and constitute a part of this specification. They are used in conjunction with the following embodiments to explain the present disclosure, but are not intended to constitute any limitation to the present disclosure. In the figures:
Fig. 1 is a perspective view of an embodiment of the humidifier in the present disclosure;
Fig. 2 is a longitudinal sectional view of the humidifier in Fig. 1;
Fig. 3 is an exploded view of the humidifier in Fig. 2;
Fig. 4 is a perspective view of an embodiment of the water-absorbing and gas-permeable component in the present disclosure;
Fig. 5 is a perspective view of the water-absorbing and gas-permeable component in Fig. 4 from another perspective;
Fig. 6 is a perspective view of an embodiment of the supporting component in the present disclosure;
Fig. 7 is a perspective view of the supporting component in Fig. 6 from another perspective;
Fig. 8 is a schematic diagram of the gas flow direction in the humidifier shown in Fig. 2 during use;
Fig. 9 is a top sectional view of the humidifier of Fig. 1, in which the gas flow direction is shown;
Fig. 10 is a longitudinal sectional view of another embodiment of the humidifier in the present disclosure;
Fig. 11 is a schematic diagram of the gas flow direction in the humidifier shown in Fig. 10 during use; and
Fig. 12 is a longitudinal sectional view of yet another embodiment of the humidifier in the present disclosure.

### Reference Numbers

10 - water tank, 11 - water storage cavity, 12 - gas inlet tube, 13 - gas outlet tube, 14 - box body, 141 - first snap fastener, 15- cover body, 151 - second snap fastener, 16 - seal, 20 - water-absorbing and gas-permeable component, 21 - gas chamber, 22 - opening, 23 - groove, 24 - annular extension, 30 - supporting component, 31 - mounting frame, 311 - inner ring, 312 - outer ring, 313 - connecting rib, 314 - guiding part, 32 - supporting frame, 321 - upper ring, 322 - supporting rib.

### DETAILED DESCRIPTION

Some embodiments of the present disclosure will be detailed below with reference to the accompanying drawings. It may be understood that the embodiments described herein are only provided to describe and explain the present disclosure, but are not intended to constitute any limitation on the present disclosure.

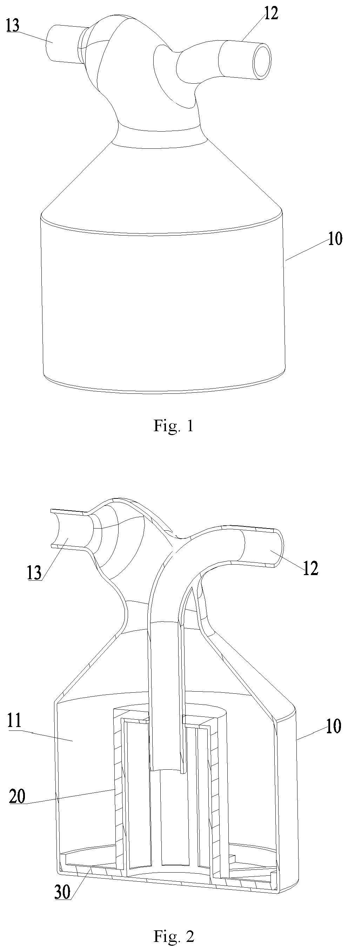

In the present disclosure, unless otherwise specified, the terms that denotes directions or orientations, such as "top", "bottom", "left", and "right", etc., usually refer to the directions or orientations as indicated in Fig. 1; and "inside" and "outside" usually refer to inside and outside with respect to the outlines of the components.

In an aspect, the present disclosure provides a humidifier, which comprises a water tank 10 having a water storage cavity 11, and a water-absorbing and gas-permeable component 20 arranged in the water storage cavity 11, wherein a part of the water-absorbing and gas-permeable component 20 is located below a water level of the water storage cavity 11; and a gas chamber 21 is defined inside the water-absorbing and gas-permeable component 20.

It can be understood that the water-absorbing and gas-permeable component 20 refers to a component that has excellent water absorbability and gas permeability. A part of the water-absorbing and gas-permeable component 20 is located below the water level of the water storage cavity 11, which means that the rest part of the water-absorbing and gas-permeable component 20 is located above the water level of the water storage cavity 11. During use, the part of the water-absorbing and gas-permeable component 20 below the water level of the water storage cavity 11 will absorb water, and then the water will be diffused to the part of the water-absorbing and gas-permeable component 20 above the water level of the water storage cavity 11; by introducing gas at a certain pressure into the water storage cavity 11, the gas will flow through the part of the water-absorbing and gas-permeable component 20 that is not immersed in water at a high speed, so that the water in the water-absorbing and gas-permeable component 20 will be evaporated and diffused into the water storage cavity 11 rapidly, thereby gas humidification is realized.

The humidifier in the present disclosure employs a cold evaporation technique in essence, which means that water molecules are evaporated into invisible ultrafine particles in size smaller than 5µm by a pneumatic device, and then are diffused into the air, so that the air is saturated with moisture. The cold-evaporated water particles are very small in diameter, and are completely invisible and will not produce water mist.

With the above technical scheme, an evaporation filter screen is formed from the water-absorbing and gas-permeable component 20 that has excellent water absorbability and gas permeability, a part of the water-absorbing and gas-permeable component 20 is immersed in water in the water storage cavity 11 to absorb water and diffuse moisture to the rest part of the water-absorbing and gas-permeable component 20, and gas entering the water storage cavity 11 at a certain pressure can flow through the part of the water-absorbing and gas-permeable component 20 that is not immersed in water at a high speed, so that the moisture in the water-absorbing and gas-permeable component 20 can be evaporated and diffused into the water storage cavity 11 rapidly, thereby realizing gas humidification; the technical scheme doesn't require heating, doesn't produce any temperature difference and condensed water, and doesn't involve the problems existing in steam humidifiers and ultrasonic humidifiers.

In the present disclosure, the water-absorbing and gas-permeable component 20 may be made of a water-absorbing and gas-permeable material, such as a fibrous material, cotton or absorbent paper.

In the present disclosure, the water tank 10 may be provided with a gas inlet tube 12 and a gas outlet tube 13, a gas outlet end of the gas inlet tube 12 is inserted into the gas chamber 21 and located above the water level of the water storage cavity 11, and the gas outlet tube 13 is in communication with the water storage cavity 11 outside the water-absorbing and gas-permeable component 20. During use, the gas entering through the gas inlet tube 12 at a certain pressure flows through the part of the water-absorbing and gas-permeable component 20 that is not immersed in water at a high speed, so that the water in the water-absorbing and gas-permeable component 20 is evaporated and diffused into the water storage cavity 11 rapidly, thereby gas humidification is realized; and the humidified gas is discharged through the gas outlet tube 13.

In the present disclosure, the water tank 10 and the water-absorbing and gas-permeable component 20 may be in any appropriate shape and size, as long as the above-mentioned function can be achieved.

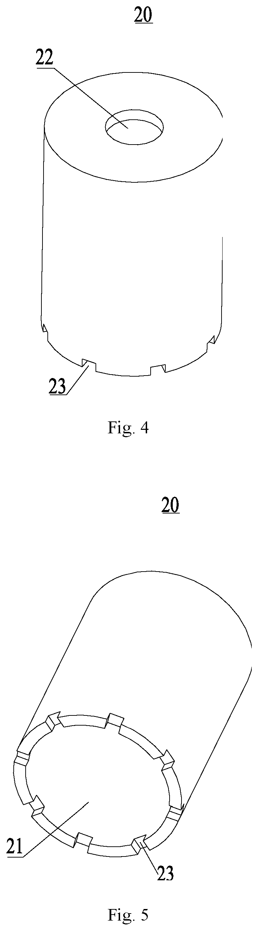

According to an embodiment of the water-absorbing and gas-permeable component 20 in the present disclosure, as shown in Figs. 4 and 5, the water-absorbing and gas-permeable component 20 may comprise a hollow cylinder, an internal cavity of the cylinder is the gas chamber 21, and a top of the cylinder is provided with an opening 22 for inserting the gas inlet tube 12.

In the present disclosure, in order to facilitate the mounting of the water-absorbing and gas-permeable component 20 in the water storage cavity 11 and prevent it from moving in the water storage cavity 11, the humidifier further comprises a supporting component 30, which is arranged in the water storage cavity 11 for supporting the water-absorbing and gas-permeable component 20.

The supporting component 30 may have any suitable structure, as long as it can be mounted in the water storage cavity 11 and support the water-absorbing and gas-permeable component 20. The structure of the supporting component 30 may be adaptively adjusted according to the shape of the water storage cavity 11 and the structure of the water-absorbing and gas-permeable component 20.

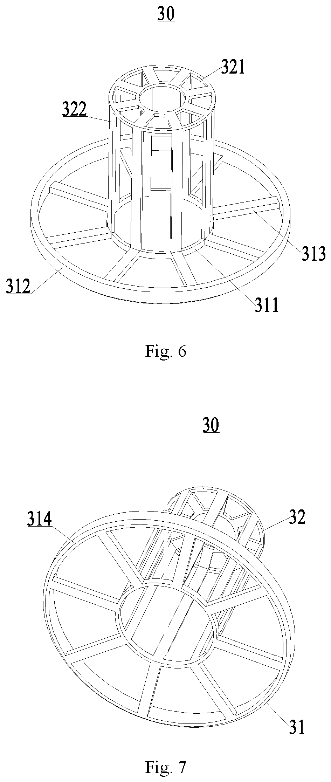

According to an embodiment of the supporting component 30 in the present disclosure, as shown in Figs. 2 and 7, the supporting component 30 may comprise a mounting frame 31 and a supporting frame 32 connected above the mounting frame 31, the supporting component 30 is mounted at a bottom of the water storage cavity 11 via the mounting frame 31, and the water-absorbing and gas-permeable component 20 is supported on the supporting frame 32.

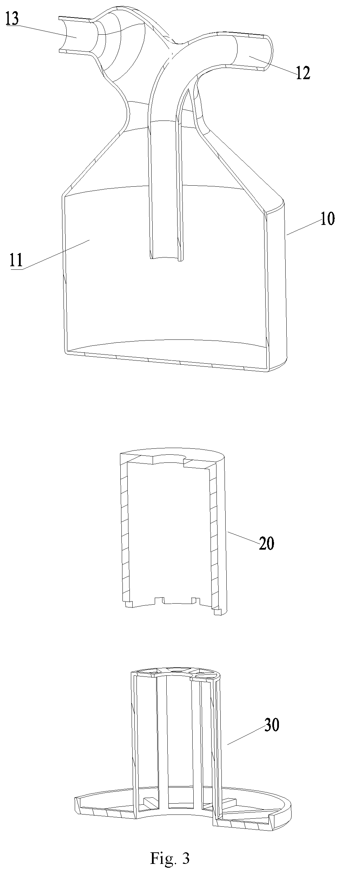

Furthermore, as shown in Figs. 2-3 and 6-7, the mounting frame 31 may be in a disk shape matching the circumferential profile of the water storage cavity 11, the supporting frame 32 is in a cylindrical shape extending upward from the mounting frame 31, and the bottom of the cylinder of the water-absorbing and gas-permeable component 20 is open and sleeved outside the supporting frame 32.

As shown in the figure, in the case that the circumferential profile of the water storage cavity 11 is circular, the mounting frame 31 is in a disk shape, the bottom surface of the mounting frame 31 is attached to the bottom wall of the water storage cavity 11, and the outer circumferential surface of the mounting frame 31 is attached to the circumferential wall of the water storage cavity 11, so that the mounting frame 31 is mounted and fixed in the water storage cavity 11.

It may be noted that the mounting frame 31 is preferably detachably mounted in the water storage cavity 11, for example, in a form-fitting manner as mentioned above, so as to facilitate the maintenance and replacement of the water-absorbing and gas-permeable component 20 and the supporting component 30. The water-absorbing and gas-permeable component 20 is preferably detachably supported on the supporting frame 32, for example, in a sleeving manner as mentioned above, so as to facilitate the maintenance and replacement of the water-absorbing and gas-permeable component 20.

Furthermore, as shown in Figs. 6 and 7, the mounting frame 31 may comprise an inner ring 311, an outer ring 312 sleeved outside the inner ring 311 at intervals, and a plurality of connecting ribs 313 connected between the inner ring 311 and the outer ring 312 at intervals along a circumferential direction; the supporting frame 32 may comprise an upper ring 321 and a lower ring that are spaced from each other in a vertical direction, and a plurality of supporting ribs 322 connected between the upper ring 321 and the lower ring and arranged at an interval in the circumferential direction, wherein the lower ring is connected to the inner ring 311, or the lower ring is the inner ring 311.

By arranging a plurality of connecting ribs 313 corresponding to the cylinder at an interval in the circumferential direction, the gas flow resistance can be decreased while the cylinder is supported, the gas flow efficiency can be ensured, and the cold evaporation effect can be improved. In addition, the supporting component 30 in the above arrangement can reduce the material consumption, reduce the manufacturing cost, and reduce the weight of the humidifier. In order to further enhance the reliability of the engagement between the cylinder and the supporting frame 32, as shown in Figs. 4 and 5, a plurality of grooves 23 for embedding the connecting ribs 313 may be arranged at the bottom end of the cylinder at an interval in the circumferential direction, and the plurality of grooves 23 are in one-to-one correspondence with the connecting ribs 313. By means of the above arrangement, the cylinder can further extend to the bottom of the water storage cavity 11.

In the above description, as shown in Fig. 2, the cylinder of the water-absorbing and gas-permeable component 20 is fully fitted outside the supporting frame 32, and the supporting frame 32 can fully support the entire cylinder, so that the water-absorbing and gas-permeable component 20 can be immersed at the bottom of the water storage cavity 11, thereby the volume of the water tank 10 can be reduced.

In addition, it can be understood that the supporting frame 32 is provided with an opening for inserting the gas inlet tube 12, and the opening corresponds to the opening 22.

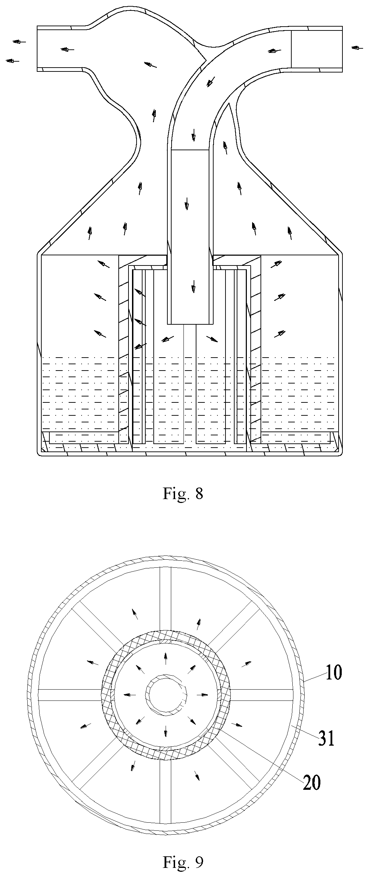

According to the present disclosure, the supporting frame 32 and the water-absorbing and gas-permeable component 20 are configured in a cylindrical shape respectively, and the gas inlet tube 12 is inserted in the axial direction, so that the gas entering the gas chamber 21 can pass through the water-absorbing and gas-permeable component and can be diffused outward in an angular range of 360 degrees into the water storage cavity 11 (see Fig. 9), thereby the gas flow is stable, free of fluctuation and turbulence, and the humidification is uniform.

In the present disclosure, in order to facilitate the mounting of the supporting component 30, the supporting component 30 is further provided with a guiding part 314 for guiding the supporting component 30 into the water storage cavity 11.

Specifically, as shown in Fig. 7, the guiding part 314 may be a chamfer formed at the outer rim of the outer ring 312.

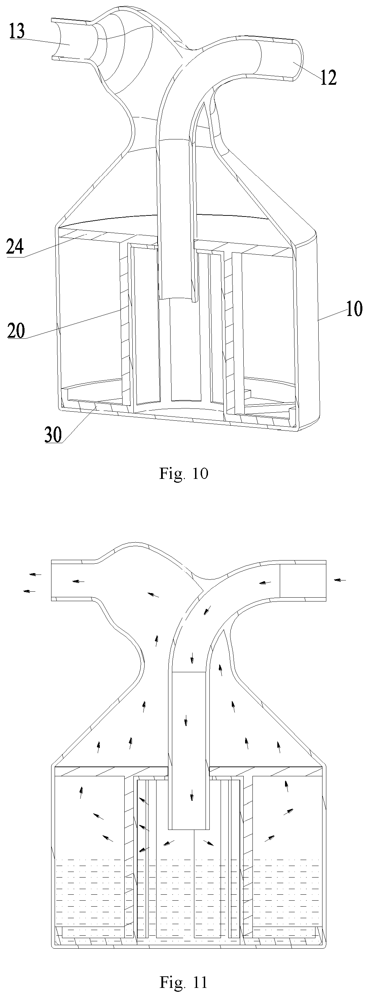

According to another embodiment of the water-absorbing and gas-permeable component 20 in the present disclosure, as shown in Fig. 10, the water-absorbing and gas-permeable component 20 may further comprise an annular extension 24 extending horizontally outward from the top circumference of the cylinder, the outer circumferential surface of the annular extension 24 is closely attached to the circumferential wall of the water storage cavity 11, and the gas outlet tube 13 is in communication with the water storage cavity 11 above the annular extension 24. By providing the annular extension 24, the water retention volume of the water-absorbing and gas-permeable component 20 can be increased, and the gas flow area can be increased. As shown in Fig. 11, the gas flow will pass through the water-absorbing and gas-permeable component 20 twice (flow through the cylinder in the first time, and flow through the annular extension 24 in the second time), thereby the humidification effect is better.

In the present disclosure, the gas inlet tube 12 and the gas outlet tube 13 may be arranged in a variety of ways, as long as the gas inlet tube 12 can introduce the gas into the gas chamber 21 of the water-absorbing and gas-permeable component 20 and the gas outlet tube 13 can discharge the humidified gas in the water storage cavity 11. In addition, depending on the application field of the humidifier, the gas inlet tube 12 and the gas outlet tube 13 may be respectively arranged to be connected with other corresponding components.

In order to lengthen the flow path of the gas in the water storage cavity 11 and further improve the humidification effect, as shown in Figs. 1-3, the gas inlet tube 12 preferably extends from the top to the bottom of the water tank 10, and the gas outlet tube 13 is preferably arranged at the top of the water tank 10, and the gas inlet end of the gas inlet tube 12 (i.e. the upper end of the gas inlet tube 12 shown in Fig. 2) is arranged opposite to the gas outlet end of the gas outlet tube 13 (i.e. the left end of the gas outlet tube 13 shown in Fig. 2). During use, as shown in Fig. 8 or Fig. 11, after the gas is introduced into the gas chamber 21 located at the lower part of the water storage cavity 11 through the gas inlet tube 12, the gas flows through the water-absorbing and gas-permeable component 20, then flows upward, and finally is discharged through the gas outlet tube 13.

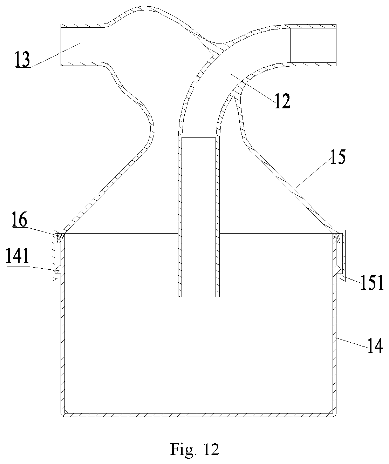

In the present disclosure, the water tank 10 may be of an integral type or a split type. To facilitate the disassembly and assembly of the water-absorbing and gas-permeable component 20 and the supporting component 30, the water tank 10 is preferably of a split type. Specifically, as shown in Fig. 12, the water tank 10 may comprise a box body 14 with a top opening, a cover body 15 detachably covering the top opening, and a seal 16 provided between the cover body 15 and the box body 14.

The seal 16 may be a seal ring made of rubber or silicone. The detachable connection between the box body 14 and the cover body 15 may be realized with any suitable structure, such as a rotary fitting structure, a snap-fit structure or an embedded cover structure in which a flange and a groove are fitted together.

According to an embodiment of the present disclosure, as shown in Fig. 12, the box body 14 and the cover body 15 are detachably connected by means of a snap-fit structure. The snap-fit structure comprises a first snap fastener 141 arranged on the box body 14 and a second snap fastener 151 arranged on the cover body 15 and adapted to the first snap fastener 141.

Furthermore, as shown in Fig. 12, the first snap fastener 141 is a protrusion arranged on the outer circumferential surface of the box body 14 and extending in the circumferential direction of the box body 14, and the second snap fastener 151 is an annular flange connected to the inner side surface of an annular wall extending in the circumferential direction of the cover body 15 at the bottom end of the cover body 15. The corresponding fitting surfaces of the protrusion and the annular flange are planar surfaces.

It may be noted that the annular wall is provided to provide a deformation space for the annular flange, so that the annular flange can be easily snap-fitted with the protrusion.

The humidifier in the present disclosure has the following advantages:
The humidifier has a simple structure, a low failure rate, high safety, high reliability and a long service life;
The water-absorbing and gas-permeable component can be replaced regularly to avoid aging and hygiene and health problems;
The humidifier doesn't require heating, and doesn't produce scale;
The humidifier doesn't produce temperature difference and condensed water;
The cold-evaporated water particles are very small in diameter, and are completely invisible and will not produce water mist;
The gas entering the gas chamber of the water-absorbing and gas-permeable component can be diffused outward in an angular range of 360 degrees, thereby the efficiency is high, there is no water mist condensation, and the humidification is more uniform.

In another aspect, the present disclosure provides a ventilation treatment apparatus, which comprises the humidifier described above.

The ventilation treatment apparatus may be an oxygen therapy apparatus or a ventilator, or the like.

For example, in a ventilator, which usually comprises a main unit, a respiratory mask, and a respiratory conduit for connecting a gas outlet of the main unit with the respiratory mask, the humidifier may be arranged in the main unit as a part of the main unit. In that case, the gas inlet tube 12 of the humidifier is in communication with the gas outlet of a fan in the main unit, and the gas outlet tube 13 of the humidifier is in communication with the gas outlet of the main unit. Alternatively, the humidifier may be arranged separately from the main unit. In that case, the gas inlet tube 12 of the humidifier may in communication with the gas outlet of the main unit, and the gas outlet tube 13 of the humidifier may be in communication with the respiratory conduit, which is to say, the humidifier may be connected between the main unit and the respiratory conduit; of course, alternatively the humidifier may be connected between the respiratory conduit and the respiratory mask.

The present invention is defined by the attached claims.

## Claims

1. A humidifier, comprising a water tank (10) having a water storage cavity (11), and a water-absorbing and gas-permeable component (20) arranged in the water storage cavity (11), wherein a part of the water-absorbing and gas-permeable component (20) is configured, in use, to be located below a water level of the water storage cavity (11); and a gas chamber (21) is defined inside the water-absorbing and gas-permeable component (20);
wherein the humidifier comprises a supporting component (30), which is arranged in the water storage cavity (11) and used for supporting the water-absorbing and gas-permeable component (20);
**characterized in that** the supporting component (30) is provided with a guiding part (314) for guiding the supporting component (30) into the water storage cavity (11).

2. The humidifier of claim 1, wherein the water tank (10) is provided with a gas inlet tube (12) and a gas outlet tube (13), a gas outlet end of the gas inlet tube (12) is inserted into the gas chamber (21) and is configured, in use, to be located above the water level of the water storage cavity (11), and the gas outlet tube (13) is in communication with the water storage cavity (11) outside the water-absorbing and gas-permeable component (20).

3. The humidifier of claim 2, wherein
the water-absorbing and gas-permeable component (20) comprises a hollow cylinder, an internal cavity of the cylinder is the gas chamber (21), and a top of the cylinder is provided with an opening (22) for inserting the gas inlet tube (12).

4. The humidifier of claim 3, wherein
the supporting component (30) comprises a mounting frame (31) and a supporting frame (32) connected above the mounting frame (31), the supporting component (30) is mounted at a bottom of the water storage cavity (11) via the mounting frame (31), and the water-absorbing and gas-permeable component (20) is supported on the supporting frame (32).

5. The humidifier of claim 4, wherein the mounting frame (31) is in a disk shape matching a circumferential profile of the water storage cavity (11), the supporting frame (32) is in a cylindrical shape extending upward from the mounting frame (31), and a bottom of the cylinder is open and sleeved outside the supporting frame (32).

6. The humidifier of claim 5, wherein the mounting frame (31) comprises an inner ring (311), an outer ring (312) sleeved outside the inner ring (311) at intervals, and a plurality of connecting ribs (313) connected between the inner ring (311) and the outer ring (312) and arranged at intervals along a circumferential direction; the supporting frame (32) comprises an upper ring (321) and a lower ring which are arranged at intervals up and down, and a plurality of supporting ribs (322) connected between the upper ring (321) and the lower ring and arranged at intervals along the circumferential direction, wherein
the lower ring is connected to the inner ring (311), or the lower ring is the inner ring (311).

7. The humidifier of claim 6, wherein the guiding part (314) is a chamfer formed at an outer rim of the outer ring (312).

8. The humidifier of claim 6 or 7, wherein a bottom end of the cylinder is provided with a plurality of grooves (23) for embedding the connecting ribs (313) at an interval in the circumferential direction, and the plurality of grooves (23) are in one-to-one correspondence with the connecting ribs (313).

9. The humidifier of any of claims 3-8, wherein the water-absorbing and gas-permeable component (20) comprises an annular extension (24) extending horizontally outward from a top peripheral edge of the cylinder, an outer circumferential surface of the annular extension (24) is closely attached to a circumferential wall of the water storage cavity (11), and the gas outlet tube (13) is in communication with the water storage cavity (11) above the annular extension (24).

10. The humidifier of any of claims 2-9, wherein
a gas inlet end of the gas inlet tube (12) and a gas outlet end of the gas outlet tube (13) are located at a top of the water tank (10) and are arranged opposite to each other, and/or
the water-absorbing and gas-permeable component (20) is made of a fibrous material, cotton or absorbent paper.

11. The humidifier of any of claims 1-10, wherein the water tank (10) comprises a box body (14) with a top opening, a cover body (15) detachably covering the top opening, and a seal (16) arranged between the cover body (15) and the box body (14).

12. A ventilation treatment apparatus, comprising the humidifier of any of claims 1-11.

## Patentansprüche

1. Befeuchter, umfassend einen Wassertank (10) mit einem Wasserspeicherhohlraum (11) und eine wasserabsorbierende und gasdurchlässige Komponente (20), die in dem Wasserspeicherhohlraum (11) angeordnet ist, wobei ein Teil der wasserabsorbierenden und gasdurchlässigen Komponente (20) dazu konfiguriert ist, sich im Gebrauch unterhalb eines Wasserspiegels des Wasserspeicherhohlraums (11) zu befinden; und eine Gaskammer (21) innerhalb der wasserabsorbierenden und gasdurchlässigen Komponente (20) definiert ist;
wobei der Befeuchter eine Stützkomponente (30) umfasst, die in dem Wasserspeicherhohlraum (11) angeordnet ist und zum Abstützen der wasserabsorbierenden und gasdurchlässigen Komponente (20) dient;
**dadurch gekennzeichnet, dass** die Stützkomponente (30) mit einem Führungsteil (314) zum Führen der Stützkomponente (30) in den Wasserspeicherhohlraum (11) versehen ist.

2. Befeuchter nach Anspruch 1, wobei der Wassertank (10) mit einem Gaseinlassrohr (12) und einem Gasauslassrohr (13) versehen ist, ein Gasauslassende des Gaseinlassrohrs (12) in die Gaskammer (21) eingeführt und dazu konfiguriert ist, sich im Gebrauch über dem Wasserspiegel des Wasserspeicherhohlraums (11) zu befinden, und das Gasauslassrohr (13) mit dem Wasserspeicherhohlraum (11) außerhalb der wasserabsorbierenden und gasdurchlässigen Komponente (20) in Verbindung steht.

3. Befeuchter nach Anspruch 2, wobei
die wasserabsorbierende und gasdurchlässige Komponente (20) einen Hohlzylinder umfasst, ein innerer Hohlraum des Zylinders die Gaskammer (21) ist, und eine Oberseite des Zylinders mit einer Öffnung (22) zum Einführen des Gaseinlassrohrs (12) versehen ist.

4. Befeuchter nach Anspruch 3, wobei
die Stützkomponente (30) einen Montagerahmen (31) und einen oberhalb des Montagerahmens (31) verbundenen Stützrahmen (32) umfasst, die Stützkomponente (30) über den Montagerahmen (31) an einem Boden des Wasserspeicherhohlraums (11) montiert ist und die wasserabsorbierende und gasdurchlässige Komponente (20) an dem Stützrahmen (32) abgestützt ist.

5. Befeuchter nach Anspruch 4, wobei der Montagerahmen (31) eine Scheibenform hat, die einem Umfangsprofil des Wasserspeicherhohlraums (11) entspricht, der Stützrahmen (32) eine zylindrische Form hat, die sich von dem Montagerahmen (31) nach oben erstreckt, und ein Boden des Zylinders offen ist und außerhalb des Stützrahmens (32) aufgesetzt ist.

6. Befeuchter nach Anspruch 5, wobei der Montagerahmen (31) einen inneren Ring (311), einen äußeren Ring (312), der in Abständen außerhalb des inneren Rings (311) aufgesetzt ist, und eine Vielzahl von Verbindungsrippen (313) umfasst, die zwischen dem inneren Ring (311) und dem äußeren Ring (312) verbunden und in Abständen entlang einer Umfangsrichtung angeordnet sind; der Stützrahmen (32) einen oberen Ring (321) und einen unteren Ring umfasst, die in Abständen nach oben und unten angeordnet sind, und eine Vielzahl von Stützrippen (322), die zwischen dem oberen Ring (321) und dem unteren Ring verbunden und in Abständen entlang der Umfangsrichtung angeordnet sind, wobei
der untere Ring mit dem inneren Ring (311) verbunden ist, oder der untere Ring der innerer Ring (311) ist.

7. Befeuchter nach Anspruch 6, wobei der Führungsteil (314) eine an einem Außenrand des äußeren Rings (312) ausgebildete Abschrägung ist.

8. Befeuchter nach Anspruch 6 oder 7, wobei ein unteres Ende des Zylinders mit einer Vielzahl von Nuten (23) zum Einbetten der Verbindungsrippen (313) in einem Abstand in der Umfangsrichtung versehen ist und die Vielzahl von Nuten (23) in eins-zu-eins-Entsprechung mit den Verbindungsrippen (313) sind.

9. Befeuchter nach einem der Ansprüche 3 bis 8, wobei die wasserabsorbierende und gasdurchlässige Komponente (20) eine ringförmige Erweiterung (24) umfasst, die sich von einer oberen Umfangskante des Zylinders horizontal nach außen erstreckt, wobei eine äußere Umfangsfläche der ringförmigen Erweiterung (24) eng an einer Umfangswand des Wasserspeicherhohlraums (11) angebracht ist und das Gasauslassrohr (13) oberhalb der ringförmigen Erweiterung (24) mit dem Wasserspeicherhohlraum (11) in Verbindung steht.

10. Befeuchter nach einem der Ansprüche 2 bis 9, wobei
ein Gaseinlassende des Gaseinlassrohrs (12) und ein Gasauslassende des Gasauslassrohrs (13) sich an der Oberseite des Wassertanks (10) befinden und einander gegenüberliegend angeordnet sind, und/oder
die wasserabsorbierende und gasdurchlässige Komponente (20) aus einem Fasermaterial, Baumwolle oder saugfähigem Papier besteht.

11. Befeuchter nach einem der Ansprüche 1 bis 10, wobei der Wassertank (10) einen Kastenkörper (14) mit einer oberen Öffnung, einen Abdeckkörper (15), der die obere Öffnung abnehmbar abdeckt, und eine Dichtung (16) umfasst, die zwischen dem Abdecckörper (15) und dem Kastenkörper (14) angeordnet ist.

12. Beatmungsbehandlungsvorrichtung, die den Befeuchter nach einem der Ansprüche 1 bis 11 umfasst.

## Revendications

1. Humidificateur, comprenant une cuve d'eau (10) ayant une cavité de stockage d'eau (11), et un composant d'absorption d'eau et perméable aux gaz (20) agencé dans la cavité de stockage d'eau (11), dans lequel une partie du composant d'absorption d'eau et perméable aux gaz (20) est configurée, durant l'utilisation, pour être localisée en-dessous d'un niveau d'eau de la cavité de stockage d'eau (11) ; et une chambre de gaz (21) est définie à l'intérieur du composant d'absorption d'eau et perméable aux gaz (20) ;
dans lequel l'humidificateur comprend un composant de support (30), qui est agencé dans la cavité de stockage d'eau (11) et utilisé pour supporter le composant d'absorption d'eau et perméable aux gaz (20) ;
**caractérisé en ce que** le composant de support (30) est pourvu d'une pièce de guidage (314) pour guider le composant de support (30) dans la cavité de stockage d'eau (11).

2. Humidificateur selon la revendication 1, dans lequel la cuve d'eau (10) est pourvue d'un tube d'orifice d'entrée de gaz (12) et d'un tube d'orifice de sortie de gaz (13), une extrémité d'orifice de sortie de gaz du tube d'orifice d'entrée de gaz (12) est insérée dans la chambre de gaz (21) et est configurée, durant l'utilisation, pour être localisée au-dessus du niveau d'eau de la cavité de stockage d'eau (11), et le tube d'orifice de sortie de gaz (13) se trouve en communication avec la cavité de stockage d'eau (11) à l'extérieur du composant d'absorption d'eau et perméable aux gaz (20).

3. Humidificateur selon la revendication 2, dans lequel
le composant d'absorption d'eau et perméable aux gaz (20) comprend un cylindre creux, une cavité interne du cylindre est la chambre de gaz (21), et un sommet du cylindre est pourvu d'une ouverture (22) pour insérer le tube d'orifice d'entrée de gaz (12).

4. Humidificateur selon la revendication 3, dans lequel
le composant de support (30) comprend un châssis de montage (31) et un châssis de support (32) raccordé au-dessus du châssis de montage (31), le composant de support (30) est monté au niveau d'un fond de la cavité de stockage d'eau (11) par l'intermédiaire du châssis de montage (31), et le composant d'absorption d'eau et perméable aux gaz (20) est supporté par le châssis de support (32).

5. Humidificateur selon la revendication 4, dans lequel le châssis de montage (31) se présente sous une forme de disque correspondant à un profil circonférentiel de la cavité de stockage d'eau (11), le châssis de support (32) se présente sous une forme cylindrique s'étendant vers le haut depuis le châssis de montage (31), et un fond du cylindre est ouvert et emmanché à l'extérieur du châssis de support (32).

6. Humidificateur selon la revendication 5, dans lequel le châssis de montage (31) comprend une bague interne (311), une bague externe (312) emmanchée à l'extérieur de la bague interne (311) par intervalles, et une pluralité de nervures de raccordement (313) raccordées entre la bague interne (311) et la bague externe (312) et agencées par intervalles le long d'une direction circonférentielle ; le châssis de support (32) comprend une bague supérieure (321) et une bague inférieure qui sont agencées par intervalles vers le haut et vers le bas, et une pluralité de nervures de support (322) raccordées entre la bague supérieure (321) et la bague inférieure et agencées par intervalles le long de la direction circonférentielle, dans lequel
la bague inférieure est raccordée à la bague interne (311), ou la bague inférieure est la bague interne (311).

7. Humidificateur selon la revendication 6, dans lequel la pièce de guidage (314) est un chanfrein formé au niveau d'un rebord externe de la bague externe (312).

8. Humidificateur selon la revendication 6 ou 7, dans lequel une extrémité inférieure du cylindre est pourvue d'une pluralité de rainures (23) pour enchâsser les nervures de raccordement (313) par intervalles dans la direction circonférentielle, et la pluralité de rainures (23) se trouvent en correspondance une-par-une avec les nervures de raccordement (313).

9. Humidificateur selon l'une quelconque des revendications 3 à 8, dans lequel le composant d'absorption d'eau et perméable aux gaz (20) comprend une extension annulaire (24) s'étendant horizontalement vers l'extérieur depuis un bord périphérique supérieur du cylindre, une surface circonférentielle externe de l'extension annulaire (24) est étroitement fixée à une paroi circonférentielle de la cavité de stockage d'eau (11), et le tube d'orifice de sortie de gaz (13) se trouve en communication avec la cavité de stockage d'eau (11) au-dessus de l'extension annulaire (24).

10. Humidificateur selon l'une quelconque des revendications 2 à 9, dans lequel
une extrémité d'orifice d'entrée de gaz du tube d'orifice d'entrée de gaz (12) et une extrémité d'orifice de sortie de gaz du tube d'orifice de sortie de gaz (13) sont localisées au niveau d'un sommet de la cuve d'eau (10) et sont agencées opposées l'une par rapport à l'autre, et/ou
le composant d'absorption d'eau et perméable aux gaz (20) est constitué d'un matériau fibreux, de coton ou de papier absorbant.

11. Humidificateur selon l'une quelconque des revendications 1 à 10, dans lequel la cuve d'eau (10) comprend un corps de boîte (14) ayant une ouverture supérieure, un corps formant couvercle (15) recouvrant de manière à pouvoir être détachée l'ouverture supérieure, et un joint (16) agencé entre le corps formant couvercle (15) et le corps de boîte (14).

12. Appareil de traitement de ventilation, comprenant l'humidificateur selon l'une quelconque des revendications 1 à 11.
